Europäisches Patentamt

European Patent Office

Office européen des brevets

(11) Veröffentlichungsnummer: **0 266 349 B1**

(12) # EUROPÄISCHE PATENTSCHRIFT

(45) Veröffentlichungstag der Patentschrift: **18.03.92**

(21) Anmeldenummer: **86904089.9**

(22) Anmeldetag: **03.07.86**

(86) Internationale Anmeldenummer:
**PCT/DE86/00278**

(87) Internationale Veröffentlichungsnummer:
**WO 87/00522 (29.01.87 87/03)**

Die Akte enthält technische Angaben, die nach
dem Eingang der Anmeldung eingereicht wurden
und die nicht in dieser Patentschrift enthalten sind.

(51) Int. Cl.5: **C07D 209/90**, C07D 487/06,
A61K 31/40, C07D 223/00,
C07D 209/00

(54) **TRICYCLISCHE INDOLDERIVATE, HERSTELLUNG UND ANWENDUNG ALS ARZNEIMITTEL.**

(30) Priorität: **15.07.85 DE 3525564**

(43) Veröffentlichungstag der Anmeldung:
**11.05.88 Patentblatt 88/19**

(45) Bekanntmachung des Hinweises auf die
Patenterteilung:
**18.03.92 Patentblatt 92/12**

(84) Benannte Vertragsstaaten:
**AT BE CH DE FR GB IT LI LU NL SE**

(56) Entgegenhaltungen:
**EP-A- 0 148 440**
**EP-A- 0 153 083**
**DE-A- 2 306 605**
**GB-A- 1 081 134**
**US-A- 3 336 307**

(73) Patentinhaber: **SCHERING AKTIENGESELL-
SCHAFT Berlin und Bergkamen
Müllerstrasse 170/178 Postfach 65 03 11
W-1000 Berlin 65(DE)**

(72) Erfinder: **HEINDL, Josef, Dr.
Eitel-Fritz-Str. 40
W-1000 Berlin 38(DE)**
Erfinder: **SCHRÖDER, Gertrud, Dr.
Theodor-Francke-Str. 3
W-1000 Berlin 42(DE)**

## Beschreibung

Die Erfindung betrifft neue Indolderivate gemäß Anspruch 1-5. deren Herstellung nach an sich bekannten Methoden und Arzneimittel auf Basis dieser Verbindungen.

Aus den Patentschriften US-A-4,110,339 und FR-A-2,471,373 sowie den Publikationen J.Med.Chem. 23, 481 (1980) und Drug.Devel. Res. 1,151 (1981) sind Verbindungen mit Ergolin-Partialstrukturen bekannt. Diese Verbindungen besitzen Dopaminagonistische Wirkung.

Weitere Verbindungen mit Ergolin-Teilstruktur werden in EP-A-29,581 beschrieben. Als Wirkungen werden eine antihypertensive, Prolactin-inhibierende und Anti-Parkinson-Aktivität angegeben. Den aus EP-A-148,440, EP-A-153,083 und EP-A-162,695 bekannten 1,3,4,5-Tetrahydrobenz[cd]-indol-4-amin-Analoga werden serotoninerge Wirkungen zugeschrieben. J.Org.Chem. 49, 4761 (1984) befaßt sich mit der Herstellung von 6-Methoxy-4-aminotetrahydrobenz[c,d]indol. Ein ähnlicher Verbindungstyp (Tetrahydroazepino [3,4,5-c,d]indole) wird in JA 43,544 und DE-A-2,306,605 erwähnt. Die pharmakologische Wirkung dieser Verbindungen wird mit ZNS-stimulierend und hypoglykämisch angegeben.

Gegenstand der vorliegenden Erfindung sind neue Tetrahydroazepino[3,4,5-c,d] indole gemäß Beispiel 1-4.

Die Salze der erfindungsgemäßen Verbindungen sind Säureadditionssalze der Immarsäure.

Die erfindungsgemäßen Verbindungen wirken Dopamin-agonistisch, d.h. sie eignen sich insbesondere für die Behandlung der Hypertonie und der Herzinsuffienz. Sie können aber ebenso gegen Prolactinhypersekretion und als Mittel gegen die Parkinson'sche Krankheit verwendet werden. Die neuen Verbindungen wirken stärker Blutdruck-senkend als vergleichbare Strukturanaloga.

Sie beeinflussen dabei die Herzfrequenz weitaus geringer und wirken zudem länger als die bekannten Verbindungen.

Zur Verwendung der erfindungsgemäßen Verbindungen als Arzneimittel werden diese in die Form eines pharmazeutischen Präparats gebracht, das neben dem Wirkstoff für die enterale oder parenterale Applikation geeignete pharmazeutische, organische oder anorganische inerte Trägermaterialien, wie z.B. Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, z.B. als Tabletten, Dragées, Suppositorien, Kapseln oder in flüssiger Form, z.B. als Lösungen, Suspensionen oder Emulsionen vorliegen. Gegebenenfalls enthalten sie darüberhinaus Hilfsstoffe wie Konservierungs-, Stabilisierungs-, Netzmittel oder Emulgatoren, Salze zur Veränderung des osmotischen Drucks oder Puffer.

Die Erfindung betrifft auch ein Verfahren zur Herstellung von Indolderivaten der Beispiele 1-4, dadurch gekennzeichnet, daß man in an sich bekannter Weise 6-Brom-4-(2-nitroethyl)-indol, 7-Benzyloxy-4-(2-nitroethyl)-indol und 7-Benzyloxy-4-(2-nitroethyl)-indol-2-carbonsäureethylester mit Fe/Eisessig reduziert und mit Formaldehyd umsetzt und gegebenenfalls die erhaltene 8-Brom-Verbindung nach Schutz der Indol-NH-Gruppe mit tert.-Butyl-dimethylchlorsilan mit tert.-Butyllithium und Borsäuretrimethylester umsetzt.

Die so erhaltenen Verbindungen werden entweder als freie Basen oder in Form ihrer Säureadditionssalze, die gewünschtenfalls durch Umsetzung mit einer physiologisch verträglichen Säure, wie Fumarsäure erhalten werden, durch Umkristallisation und/oder Chromatographie gereinigt.

Die Umsetzung der Ausgangsverbindungen gemäß den Beispielen 1-4 mit Fe-Pulver/Eisessig und anschließend mit Formaldehyd verläuft in optimaler Weise unter den Bedingungen, wie sie in den Beispielen angegeben werden. Selbstverständlich können der Temperaturbereich nach oben oder unten ausgedehnt, das Lösungsmittel durch ein chemisch äquivalentes Lösungsmittel ersetzt und auch die Reaktionszeit variiert werden.

## Referenzbeispiel 1

### 4-Dimethylamino-7-hydroxy-1,3,4,5-tetrahydrobenz-[c,d]indol, Hydrogenfumarat

A. 7-Brom-1-tert.-butyldimethylsilyl-4-dimethylamino-1,3,4,5-tetrahydrobenz[c,d]indol

Eine Lösung von 2,5 ml Diisopropylamin in 11 ml absolutem Tetrahydrofuran wird unter Argonatmosphäre, Rühren und Eiskühlung tropfenweise mit 7,65 ml n-Buthyllithium (15%ig in Hexan) versetzt, auf -20°C gekühlt und eine Lösung von 2,4 g 7-Brom-4-dimethylamino-1,3,4,5-tetrahydrobenz-[c,d]indol in 30 ml absolutem Tetrahydrofuran zugetropft. Die Mischung wird 20 Minuten bei -20°C gerührt und bei dieser Temperatur tropfenweise eine Lösung von 2,6 g tert.-Butyldimethylsilylchlorid in 5 ml absolutem Tetrahydrofuran hinzugefügt. Dann läßt man die Reaktionsmischung auf Raumtemperatur kommen, rührt noch 17 Stunden, gießt sie in 150 ml 10%ige Natriumcarbonatlösung und schüttelt 3 x mit je 50 ml Ethylacetat aus. Die organischen Phasen werden über Natriumsulfat getrocknet, eingeengt und der Rückstand an Kieselgel mit Dichlormethan + 5 - 10% Isopropanol als Elutionsmittel chromatographiert. Man erhält so 2,93 g 7-Brom-1-tert.-butyldimethylsilyl-4-dimethylamino-1,3,4,5-tetrahydrobenz[c,d]indol als Öl.

B. 4-Dimethylamino-7-hydroxy-1,3,4,5-tetrahydrobenz[c,d]indol, Hydrogenfumarat

Eine Lösung von 1,2 g 7-Brom-1-tert.-butyldimethylsilyl-4-dimethylamino-1,3,4,5-tetrahydrobenz[c,d]indol in 120 ml absolutem Tetrahydrofuran wird unter Rühren und Argonatmosphäre bei -90°C tropfenweise mit 4,2 ml tert.-Butyllithium (2molare Lösung in Pentan) versetzt und 5 Minuten bei dieser Temperatur gerührt. Dann wird bei -75°C eine Lösung von 733 mg Borsäuretrimethylester in 4,5 ml absolutem Tetrahydrofuran zugetropft, 15 Minuten bei dieser Temperatur und 1 1/2 Stunden bei Raumtemperatur gerührt. Die Mischung wird auf -5°C gekühlt, nacheinander mit 0,77 ml Eisessig, einer Mischung aus 1 ml 30 %igem Wasserstoffperoxid und 1 ml Wasser versetzt und 20 Minuten bei 0°C gerührt. Die Reaktionsmischung wird in 350 ml Eiswasser gegeben, mit konzentrierter Ammoniaklösung bis zur alkalischen Reaktion versetzt und 3 x mit je 100 ml Dichlormethan extrahiert. Die organische Phase wird mit 10 %iger Ammoniumeisensulfatlösung gewaschen, getrocknet ($Na_2SO_4$) und eingeengt. Der Rückstand wird an Kieselgel mit Dichlormethan/Isopropanol/konz. Ammoniaklösung = 95/5/1 als Elutionsmittel chromatographiert. Das eingeengte Eluat wird in 6,5 ml Trifluoressigsäure und 0,65 ml Wasser 15 Minuten bei 40°C gerührt, in 10 ml Eiswasser gegeben und unter Eiskühlung mit konzentrierter Ammoniaklösung bis zur alkalischen Reaktion versetzt. Es wird 3 x mit je 10 ml Dichlormethan extrahiert, die organische Phase getrocknet ($Na_2SO_4$), eingeengt und der Rückstand an Kieselgel mit Dichlormethan/Isopropanol/konz. Ammoniaklösung = 95/5/1 als Elutionsmittel chromatographiert. Das eingeengte Eluat wird in wenig Ethanol, mit ethanolischer Fumarsäurelösung und Diethylether versetzt und der beim Kühlen erhaltene Niederschlag abgesaugt. Man erhält so 180 mg 4-Dimethylamino-7-hydroxy-1,3,4,5-tetrahydrobenz[c,d]indol, Hydrogenfumarat vom Schmelzpunkt 173 - 181°C (Zersetzung).

Referenzbeispiel 2

8-Hydroxy-1,3,4,5-tetrahydrobenz[c,d,]indol-4-amin, Fumarat

A. 7-Benzyloxy-4-(2-nitrovinyl)-indol

Eine suspension von 28,7 g 7-Benzyloxy-4-formylindol (EP 82 103 106.9) in 185 ml Methanol wird mit 6,7 ml Nitromethan, 2,9 g Methylammoniumchlorid und 2,9 g Kaliumacetat versetzt und die Mischung 3 Tage bei Raumtemperatur gerührt. Der orangefarbene Niederschlag wird abgesaugt und man erhält so 30,8 g 7-Benzyloxy-4-(2-nitrovinyl)-indol vom Schmelzpunkt 159 - 161°C.

B. 7-Benzyloxy-4-(2-nitroethyl)-indol

32,5 g 7-Benzyloxy-4-(2-nitrovinyl)-indol werden in einer Mischung aus 1400 ml Chloroform und 280 ml Isopropanol gelöst, mit 221 g Kieselgel und 16,7 g Natriumborhydrid versetzt und 24 Stunden bei Raumtemperatur gerührt. Danach wird die Reaktionsmischung unter Eiskühlung mit Eisessig bis pH 6 angesäuert, filtriert, der Filterrückstand mit Dichlormethan gewaschen, die vereinigten Filtrate mit gesättigter Kochsalzlösung gewaschen, getrocknet ($Na_2SO_4$) und eingeengt. Der Rückstand wird an Kieselgel mit Hexan/Ethylacetat = 2/1 als Elutionsmittel chromatographiert. Das eingeengte Eluat wird aus Toluol/Hexan kristallisiert und man erhält so 24,8 g 7-Benzyloxy-4-(2-nitroethyl)-indol Schmelzpunkt 82 - 83°C.

C. 8-Benzyloxy-4-nitro-1,5-dihydrobenz[c,d]indol

5,1 ml Phosphoroxytrichlorid werden unter Rühren bei 0 - 5°C in 30 ml Dimethylformamid getropft und die Mischung bei dieser Temperatur tropfenweise mit einer Lösung von 15 g 7-Benzyloxy-4-(2-nitroethyl)-indol in 120 ml Dimethylformamid versetzt. Nach zweistündigem Rühren bei Raumtemperatur wird die Reaktionsmischung in 250 ml gesättigte Natriumcarbonatlösung gegossen, 3 Stunden nachgerührt und der dunkelrote Niederschlag abgesaugt. Man erhält so 15,1 g 8-Benzyloxy-4-nitro-1,5-dihydrobenz[c,d]indol vom Schmelzpunkt 170°C (Zersetzung).

D. 8-Benzyloxy-4-nitro-1,3,4,5-tetrahydrobenz[c,d]-indol

5,7 g 8-Benzyloxy-4-nitro-1,5-dihydrobenz[c,d]-indol werden in einer Mischung aus 235 ml Chloroform und 47 ml Isopropanol gelöst, mit 37,5 g Kieselgel und 2,9 g Natriumborhydrid versetzt und 3 Stunden bei Raumtemperatur gerührt. Danach werden unter Eiskühlung 30 ml 2 n Salzsäure zugetropft, die Reaktionsmischung filtriert, der Filterrückstand mit Dichlormethan gewaschen, die vereinigten Filtrate mit gesättigter Kochsalzlösung gewaschen, getrocknet ($Na_2SO_4$) und eingeengt. Der Rückstand wird an Kieselgel mit Dichlormethan als Elutionsmittel chromatographiert. Nach Einengen des Eluats werden 3,8 g 8-Benzyloxy-4-nitro-1,3,4,5-tetrahydrobenz[c,d]indol vom Schmelzpunkt 127 - 128°C erhalten.

E. 8-Benzyloxy-1,3,4,5-tetrahydrobenz[c,d]indol-4-amin

Zu einer Suspension von 33 g Eisenpulver in 130 ml Eisessig wird unter Rühren bei 80°C eine Lösung von 16,3 g 8-Benzyloxy-4-nitro-1,3,4,5-tetrahydrobenz[c,d]indol getropft. Nach beendeter Zugabe wird die Mischung noch 3 Stunden bei 80°C gerührt, nach Abkühlen auf Raumtemperatur bis zur alkalischen Reaktion mit einer gesättigten Lösung von Natriumcarbonat versetzt und der Niederschlag über Kieselgur abgesaugt. Das Filtrat wird mit Diethylether ausgeschüttelt, die organischen Phasen getrocknet (Na$_2$SO$_4$) und eingeengt. Der Rückstand wird an Kieselgel mit Ethylacetat/Methanol/konz. Ammoniaklösung = 75/23/2 chromatographiert. Man erhält so 13,1 g 8-Benzyloxy-1,3,4,5-tetrahydrobenz[c,d]indol-4-amin als braunes Öl.

F. 8-Hydroxy-1,3,4,5-tetrahydrobenz[c,d]indol-4-amin, Fumarat

Eine Lösung von 750 mg 8-Benzyloxy-1,3,4,5-tetrahydrobenz [c,d]indol-4-amin in 40 ml Tetrahydrofuran wird in Gegenwart von 100 mg 10%igem Palladium-Katalysator auf Aktiv-Kohle unter Schütteln 6 Stunden bei Normaldruck hydriert. Die Reaktionsmischung wird filtriert, das Filtrat eingeengt, der Rückstand in wenig Ethanol gelöst, mit ethanolischer Fumarsäurelösung und Diethylester versetzt und der Niederschlag abgesaugt. Man erhält so 500 mg 8-Hydroxy-1,3,4,5-tetrabenz[c,d]indol-4-amin, Fumarat vom Schmelzpunkt 220°C (Zersetzung).

Beispiel 1

8-Brom-3,4,5,6-tetrahydro-1H-azepino[3,4,5-c,d]-indol, Fumarat

A. 4-(2-Aminoethyl)-6-bromindol

Unter den Bedingungen des Referenz-Beispiels 2 E werden 10 g 6-Brom-4-(2-nitroethyl)-indol umgesetzt, aufbereitet und man erhält so 7 g 4-(2-Aminoethyl)-6-bromindol als Öl.

B. 8-Brom-3,4,5,6-tetrahydro-1H-azepino[3,4,5-c,d]-indol, Fumarat

5 g 4-(2-Aminoethyl)-6-bromindol werden in 100 ml 1 n Salzsäure gelöst, mit 10 %iger Natriumhydrogencarbonatlösung ein pH von 6,5 eingestellt, mit 1,56 ml 37 %iger Formaldehydlösung versetzt und 3 1/2 Stunden bei Raumtemperatur gerührt. Danach wird bis zur alkalischen Reaktion 25 %ige Ammoniaklösung hinzugefügt, 3 x mit Ethylacetat ausgeschüttelt, die organischen Phasen getrocknet (Na$_2$SO$_4$) und eingeengt. Der Rückstand wird an Kieselgel mit Ethylacetat/Methanol/25%iger Ammoniaklösung = 75/23/2 als Elutionsmittel chromatographiert. Das eingeengte Eluat wird in wenig Ethanol gelöst, mit ethanolischer Fumarsäurelösung und Diethylether versetzt und der Niederschlag abgesaugt. Man erhält so 3,5 g 8-Brom-3,4,5,6-tetrahydro-1H-azepino-[3,4,5-c,d]indol, Fumarat vom Schmelzpunkt 203 - 206°C.

Beispiel 2

9-Hydroxy-3,4,5,6-tetrahydro-1H-azepino[3,4,5-c,d]-indol, Fumarat

A. 4-(2-Aminoethyl)-7-benzyloxyindol

Unter den Bedingungen des Referenz-Beispiels 2 E werden 4,9 g 7-Benzyloxy-4-(2-nitroethyl)-indol umgesetzt, aufbereitet und man erhält so 4,5 g 4(2-Aminoethyl)-7-benzyloxyindol als Öl.

B. 9-Benzyloxy-3,4,5,6-tetrahydro-1H-azepino[3,4,5-c,d]indol

Unter den Bedingungen des Beispiels 1 B werden 3,3 g 4-(2-Aminoethyl)-7-benzyloxyindol mit 0,92 ml 37 %iger Formaldehydlösung umgesetzt, aufbereitet und man erhält so 1,4 g 9-Benzyloxy-3,4,5,6-tetrahydro-1H-azepino[3,4,5-c,d]indol vom Schmelzpunkt 198 - 199°C.

C. 9-Hydroxy-3,4,5,6-tetrahydro-1H-azepino[3,4,5-c,d]indol, Fumarat

Unter den Bedingungen des Referenz-Beispiels 2 F werden 350 mg 9-Benzyloxy-3,4,5,6-tetrahydro-1H-azepino[3,4,5-c,d]indol hydriert, aufbereitet und man erhält so 40 mg 9-Hydroxy-3,4,5,6-tetrahydro-1H-azepino[3,4,5-c,d]indol, Fumarat, das sich ab 240°C zersetzt.

Beispiel 3

9-Hydroxy-4-methyl-3,4,5,6-tetrahydro-1H-azepino-[3,4,5-c,d]indol-2-carbonsäureethylester, Hydrogenfumarat

A. 7-Benzyloxy-4-(2-nitrovinyl)-indol-2-carbonsäureethylester

Eine Lösung von 5 g 7-Benzyloxy-4-formylindol-2-carbonsäureethylester (EP 82103106.9) in 50 ml Nitromethan wird mit 1,62g Ammoniumacetat versetzt und 3 Stunden unter Rückfluß erhitzt. Danach wird das Nitromethan im Vakuum entfernt, der Rückstand in Wasser gegeben und mit Ethylacetat extrahiert. Die organischen Phasen werden getrocknet (Na$_2$SO$_4$) eingeengt

und der Rückstand aus Ethylacetat umkristallisiert. Man erhält so 3,5 g 7-Benzyloxy-4-(2-nitrovinyl)-indol-2-carbonsäureethylester vom Schmelzpunkt 163 - 165°C.

B.   7-Benzyloxy-4-(2-nitroethyl)-indol-2-carbonsäureethylester

Unter den Bedingungen des Referenz-Beispiels 2 B werden 5 g 7-Benzyloxy-4-(2-nitrovinyl)-indol-2-carbonsäureethylester mit 2 g Natriumborhydrid umgesetzt, aufbereitet und man erhält so 3,7 g 7-Benzyloxy-4-(2-nitroethyl)-indol-2-carbonsäureethylester vom Schmelzpunkt 99 - 101°C.

C. 7-Benzyloxy-4-(2-aminoethyl)-indol-2-carbonsäureethylester

Unter den Bedingungen des Referenz-Beispiels 2 E werden 6 g 7-Benzyloxy-4-(2-nitroethyl)-indol-2-carbonsäureethylester umgesetzt, aufbereitet und man erhält so 5,4 g 7-Benzyloxy-4-(2-aminoethyl)-indol-2-carbonsäureethylester vom Schmelzpunkt 135-140°C.

D.   9-Benzyloxy-4-methyl-3,4,5,6-tetrahydro-1H-azepino[3,4,5-c,d]indol-2-carbonsäureethylester

5,1 g 7-Benzyloxy-4-(2-aminoethyl)-indol-2-carbonsäureethylester werden in 148 ml 1 n Natriumdihydrogenphosphitlösung und 148 ml Dioxan gelöst, mit 11,2 ml 37 %iger Formaldehydlösung versetzt und die Mischung 9 1/2 Stunden unter Rühren auf 60°C erhitzt. Die Reaktionsmischung wird bis zur alkalischen Reaktion mit 10%iger Natriumcarbonatlösung versetzt, 3 x mit Ethylacetat extrahiert, die organischen Phasen getrocknet und eingeengt. Der Rückstand wird an Kieselgel mit Ethylacetat/Methanol/25 %iger Ammoniaklösung = 75/23/2 als Elutionsmittel chromatographiert. Es werden so 1,3 g 9-Benzyloxy-4-methyl-3,4,5,6-tetrahydro-1H-azepino[3,4,5-c,d]indol-2-carbonsäureethylester als Fraktion I (Schmelzpunkt 100-103°C) neben 2,5g 9-Benzyloxy-4-(2-dimethylaminoethyl)-indol-2-carbonsäureethylester (Schmelzpunkt 76-78°C) als Fraktion II erhalten.

E.   9-Hydroxy-4-methyl-3,4,5,6-tetrahydro-1H-azepino[3,4,5-c,d]indol-2-carbonsäureethylester, Hydrogenfumarat

Eine Lösung von 700 mg 9-Benzyloxy-4-methyl-3,4,5,6-tetrahydro-1 H-azepino[3,4,5-c,d]-indol-2-carbonsäureethylester in 35 ml Ethanol wird in Gegenwart von 70 mg 10%igem Palladium-Katalysator auf Aktiv-Kohle unter Schütteln 6 Stunden bei Normaldruck hydriert. Die Reaktionsmischung wird filtriert, das Filtrat eingeengt und der Rückstand an Kieselgel mit Aceton/Triäthylamin = 20 : 1 als Elutionsmittel chromtographiert. Das eingeengte Eluat wird in wenig Ethanol gelöst, mit ethanolischer Fumarsäurelösung und Diethylether versetzt und der beim Kühlen erhaltene Niederschlag abgesaugt. Man erhält so 250 mg 9-Hydroxy-4-methyl-3,4,5,6-tetrahydro-1H-azepino[3,4,5-c,d]-indol-2-carbonsäureethylester, Hydrogenfumarat vom Schmelzpunkt 241 - 243°C.

Beispiel 4

3,4,5,6-Tetrahydro-1H-azepino[3,4,5-c,d]indol, Fumarat und 8-Hydroxy-3,4,5,6-tetrahydro-1H-azepino[3,4,5-c,d]indol, Fumarat

A.   8-Brom-1-tert.-butyldimethylsilyl-3,4,5,6-tetrahydro-1H-azepino[3,4,5-c,d]indol

Unter den Bedingungen des Referenz-Beispiels 1 A werden 5,3 g 8-Brom-3,4,5,6-tetrahydro-1H-azepino[3,4,5-c,d]indol mit 12,6 g tert.-Butyldimethylsilylchlorid umgesetzt, aufbereitet und an Kieselgel mit Ethylacetat/Methanol/25 %igem Ammoniak = 75/23/2 als Elutionsmittel chromatographiert. Man erhält so 3,5 g 8-Brom-1-tert.-butyldimethylsilyl-3,4,5,6-tetrahydro-1H-azepino-[3,4,5-c,d]indol vom Schmelzpunkt 132 - 135°C.

B.   3,4,5,6-Tetrahydro-1H-azepino[3,4,5-c,d]indol, Fumarat und 8-Hydroxy-3,4,5,6-tetrahydro-1H-azepino[3,4,5-c,d] indol, Fumarat

Unter den Bedingungen des Referenz-Beispiels 1 B werden 1,75 g 8-Brom-1-tert.-butyldimethylsilyl-3,4,5,6-tetrahydro-1H-azepino-[3,4,5-c,d]indol mit 7,61 ml tert.- Butyllithium (2,3 molar in Pentan) und 1,73 ml Borsäuretrimethylester umgesetzt, aufbereitet und an Kieselgel mit Dichlormethan/Methanol/ 25 %iger Ammoniaklösung = 34/6/1 und 15/5/1 als Elutionsmittel chromatographiert. Als Fraktion I werden 220 mg 3,4,5,6-Tetrahydro-1H-azepino[3,4,5-c,d]indol, Fumarat vom Schmelzpunkt 190 - 191°C (Zersetzung) und als Fraktion II 85 mg 8-Hydroxy-3,4,5,6-tetrahydro-1H-azepino[3,4,5-c,d]indol, Fumarat, das sich ab 190°C zersetzt, erhalten.

**Patentansprüche**

1. 8-Brom-3,4,5,6-tetrahydro-1H-azepino[3,4,5-c,d]-indol, Fumarat.

2. 9-Hydroxy-3,4,5,6-tetrahydro-1H-azepino[3,4,5-c,d]-indol, Fumarat.

3. 9-Hydroxy-4-methyl-3,4,5,6-tetrahydro-1H-azepino[3,4,5-c,d]-indol-2-carbonsäureethylester, Hydrogenfumarat.

4. 3,4,5,6-Tetrahydro-1H-azepino[3,4,5-c,d]-indol, Fumarat.

5. 8-Hydroxy-3,4,5,6-tetrahydro-1H-azepino[3,4,5-c,d]-indol, Fumarat.

6. Verfahren zur Herstellung von Indolderivaten gemäß Anspruch 1-5, dadurch gekennzeichnet, daß man in an sich bekannter Weise 6-Brom-4-(2-nitroethyl)-indol, 7-Benzyloxy-4-(2-nitroethyl)-indol und 7-Benzyloxy-4-(2-nitroethyl)-indol-2-carbonsäureethylester mit Fe/Eisessig reduziert und mit Formaldehyd umsetzt und gegebenenfalls die erhaltene 8-Bromverbindung nach Schutz der Indol-NH-Gruppe mit tert.-Butyldimethylchlorsilan mit tert.-Butyllithium und Borsäuretrimethylester umsetzt.

7. Arzneimittel enthaltend eine Verbindung oder ein Verbindungsgemisch gemäß Anspruch 1-5.

## Claims

1. 8-Bromo-3,4,5,6-tetrahydro-1H-azepino[3,4,5-c,d]-indole, fumarate.

2. 9-Hydroxy-3,4,5,6-tetrahydro-1H-azepino[3,4,5-c,d]-indole, fumarate.

3. 9-Hydroxy-4-methyl-3,4,5,6-tetrahydro-1H-azepino[3,4,5-c,d]-indole-2-carboxylic acid ethyl ester, hydrogen fumarate.

4. 3,4,5,6-Tetrahydro-1H-azepino[3,4,5-c,d]-indole, fumarate.

5. 8-Hydroxy-3,4,5,6-tetrahydro-1H-azepino[3,4,5-c,d]-indole, fumarate.

6. Process for the preparation of indole derivatives according to claims 1 to 5, characterised in that, in a manner known per se, 6-bromo-4-(2-nitroethyl)-indole, 7-benzyloxy-4-(2-nitroethyl)-indole and 7-benzyloxy-4-(2-nitroethyl)-indole-2-carboxylic acid ethyl ester are reduced with Fe/glacial acetic acid and reacted with formaldehyde and, if desired, the resulting 8-bromo compound, after protection of the indole-NH group with tert-butyldimethylchlorosilane, is reacted with tert-butyllithium and boric acid trimethyl ester.

7. Medicament containing a compound or a compound mixture according to claim 1 to 5.

## Revendications

1. Fumarate du bromo-8 tétrahydro-3,4,5,6 1H-azépino[3,4,5-cd]indole.

2. Fumarate de l'hydroxy-9 tétrahydro-3,4,5,6 1H-azépino[3,4,5-cd]indole.

3. Hydrogénofumarate de l'hydroxy-9 méthyl-4 tétrahydro-3,4,5,6 1H-azépino[3,4,5-cd]indole-carboxylate-2 d'éthyle.

4. Fumarate du tétrahydro-3,4,5,6 1H-azépino-[3,4,5-cd]indole.

5. Fumarate de l'hydroxy-8 tétrahydro-3,4,5,6 1H-azépino[3,4,5-cd]indole.

6. Procédé pour préparer des dérivés de l'indole suivant l'une quelconque des revendications 1 à 5, procédé caractérisé en ce que, en opérant de manière connue, on réduit au moyen de fer et d'acide acétique glacial le bromo-6 (nitro-2 éthyl)-4 indole, le benzyloxy-7 (nitro-2 éthyl)-4 indole et le benzyloxy-7 (nitro-2 éthyl)-4 indole-carboxylate-2 d'éthyle, on fait réagir avec le formaldéhyde, et éventuellement on fait réagir le composé bromé en 8 obtenu, après avoir protégé le radical NH de l'indole par du tert-butyl-diméthyl-chlorosilane, avec du tert-butyl-lithium et du borate de triméthyle.

7. Médicaments contenant un composé ou un mélange de composés selon l'une quelconque des revendications 1 à 5.